# EUROPEAN PATENT APPLICATION

(11) **EP 4 847 688 A1**
(43) Date of publication of application: **02.12.2026**
(21) Application number: 25179360.0
(22) Date of filing: 28.05.2025
(51) Int. Cl.: D06M 16/00

(54) **METHOD AND APPARATUS FOR MULTILAYER COATING OF MEDICAL TEXTILE SUBSTRATES**

(71) Applicant: Paul Hartmann AG, 89522 Heidenheim (DE)
(72) Inventor: SCHMIDT, Christian, 73466 Lauchheim (DE)
(74) Representative: Paul Hartmann AG Patents & Licensing

(57) **Abstract**

The present disclosure provides a method for coating a medical textile substrate web with at least two layers of coating, particularly an antimicrobial effective coating. The method includes providing the textile substrate web in a donor device, applying coating solutions A and B comprising different coating molecules to sections of the web, removing excessive residues, optionally drying the web, and collecting it in a receiving device. The textile substrate web is continuously moved from the donor device to the receiving device. An apparatus for coating a textile substrate web is also provided, comprising a donor device, coating baths or spray-coating devices, rinsing baths or flushing devices, a drying device, a receiving device, and optionally diverter rollers or bars. The apparatus is configured to perform the coating method. Additionally, a coated textile substrate web produced by the method and a medical dressing or bandage comprising a textile layer cut from the coated web are disclosed.

## Description

### FIELD OF INVENTION

The present disclosure relates to methods and apparatuses for coating textile substrates, and more particularly to a method and apparatus for applying multiple layers of antimicrobial coatings to a continuously moving textile substrate web.

### BACKGROUND

Textile materials are widely used in various applications, including medical and healthcare settings. Antimicrobial coatings on textiles can provide protection against harmful microorganisms and improve hygiene. Applying such coatings to textile substrates presents challenges in achieving uniform coverage, durability, and maintaining the fabric's original properties.

One approach to coating textiles involves immersing the fabric in a solution containing the desired coating material. US 5409739 A describes a process for producing a crosslinked monolayer coating on a substrate using block copolymers. This method involves introducing a block copolymer into a selective solvent, contacting the substrate with the resulting semi-solution, and crosslinking the coating. While this approach can create thin, durable coatings, it may not be suitable for applying multiple layers or for continuous processing of large textile webs.

WO 2016/011944 A1 discloses a multi-layer coating system comprising two different coating compositions applied sequentially. The system aims to provide good adhesion and resistance properties. However, this method focuses on coating rigid substrates like plastics for electronic devices and may not be directly applicable to flexible textile materials or continuous web processing.

WO 2017/191110 A1 discloses a method for coating a substrate with alternating layers of polyelectrolytes. The document describes using this technique to create antimicrobial coatings on medical devices and implants. The method as described does not disclose coating of flexible textile substrates used in medical dressings or bandages. Also, while this approach allows for the creation of multilayer coatings with controlled thickness, it may face challenges in scaling up for continuous processing of large textile webs at industrial speeds.

US 4093757 A presents a method for coating a substrate with a monomolecular layer of organic molecules using a Langmuir-Blodgett technique. This approach allows for precise control of layer thickness but may be limited in terms of processing speed and scalability for large textile webs.

US 2020/0223201 A1 describes a method for treating substrates using atmospheric plasma to affix biomaterials like silk or wool polypeptides to textile surfaces. While this technique offers advantages in terms of environmental friendliness, it may not be suitable for applying multiple distinct layers of coating materials.

US 6762243 B2 discloses a multilayer coating system using polyurethane compositions. This system provides flexibility and durability but may not offer the precision required for creating alternating layers of different coating materials on a textile substrate.

CN 106637925 A presents a method for preparing ultraviolet-resistant cotton fabric using a single-molecule layer of graphene oxide. While this approach demonstrates the potential for thin, functional coatings on textiles, it is limited to a single layer and specific coating material.

Existing coating methods for textiles face several significant challenges that limit their effectiveness, particularly for antimicrobial applications in medical and healthcare settings:
1. Difficulty in applying multiple distinct layers of coating materials - Current methods typically focus on single-layer applications and struggle to create well-defined, alternating layers of different coating materials with precise interfaces between them. This limitation restricts the ability to create coatings with complementary properties or synergistic antimicrobial effects.
2. Limited control over layer thickness and uniformity - Conventional coating techniques often result in inconsistent layer thickness across the textile substrate, with variations that can exceed 50% between different areas of the same substrate. This non-uniformity leads to unpredictable performance characteristics and potential weak points in antimicrobial protection.
3. Challenges in continuous processing of large textile webs - Many existing coating methods are batch processes or limited to small-scale applications, making them impractical for industrial-scale production of coated textiles. Continuous web processing at speeds sufficient for commercial viability (typically 5-20 meters per minute) presents significant technical hurdles for maintaining coating quality and consistency.
4. Potential alteration of textile properties like flexibility and breathability - Many coating processes significantly compromise the inherent properties of the textile substrate, reducing breathability by for example up to 40-60%, increasing stiffness, and altering tactile characteristics. These changes can render the coated textiles unsuitable for applications requiring comfort and flexibility, such as medical garments or wound dressings.
5. Limited scalability for industrial production - Techniques such as Langmuir-Blodgett deposition or plasma treatment offer excellent control but are difficult to scale up economically. The capital investment, energy consumption, and operational complexity of these methods often make them prohibitively expensive for large-scale textile production, with costs typically 3-5 times higher than conventional textile finishing processes.
6. Inadequate durability of coatings, especially for medical applications - Many antimicrobial coatings show significant degradation after relatively few washing cycles (typically losing 30-50% efficacy after 5-10 washes) or during sterilization processes. This limited durability restricts their use in medical textiles that require repeated cleaning or sterilization while maintaining consistent antimicrobial performance throughout their service life.
7. Insufficient or inconsistent antimicrobial activity, particularly after repeated washing or extended use - Single-layer antimicrobial coatings often exhibit diminishing efficacy over time, with significant reductions in antimicrobial performance (measured by log reduction values against test organisms) after exposure to moisture, bodily fluids, or cleaning agents. This inconsistency creates unpredictable protection levels and potential failure points in critical applications where sustained antimicrobial activity is essential.

Considering these limitations, there is a need for an improved method and apparatus for applying multiple layers of functional coatings to textile substrates in a continuous, scalable process while maintaining the desirable properties of the base material.

### DETAILED DESCRIPTION

The present invention solves the problems mentioned above by providing a method and apparatus for coating medical textile substrate webs with multilayer coatings, particularly antimicrobial multilayer coatings. The invention enables the application of alternating layers quickly, easily, and cost-effectively on an industrial scale.

It was surprisingly found that alternating layers of preferably antimicrobial active coatings can stably be formed on a textile substrate web. This discovery may provide several advantages for creating functional coatings on medical textiles.

The method for coating a medical textile substrate web with at least two layers of coating involves providing the textile substrate web in a donor device and continuously moving the textile substrate web from the donor device to a receiving device. The process includes applying a coating solution A comprising coating molecules A to a section of the textile substrate web, followed by removing excessive residues of coating solution A. Subsequently, a coating solution B comprising coating molecules B is applied to the section of the textile substrate web, followed by removing excessive residues of coating solution B. The coating molecules A and coating molecules B are different. Optionally, the section of the textile substrate web may be dried using a drying device. Finally, the coated textile substrate web is collected in the receiving device.

An apparatus for coating a textile substrate web comprises a donor device for providing the textile substrate web, a first coating bath or spray-coating device, a first rinsing bath or flushing device, a second coating bath or spray-coating device, a second rinsing bath or flushing device, a drying device, and a receiving device for collecting the coated textile substrate web.

The apparatus may optionally include a plurality of diverter rollers or diverter bars for guiding the textile substrate web through the process.

The invention provides a versatile and efficient method for applying multilayer coatings to medical textile substrates. By utilizing different coating solutions and implementing a continuous process, the method allows for the creation of complex coating structures with precise control over layer composition and thickness. The removal of excessive residues between coating applications ensures clean and well-defined layers, while the optional drying step can be used to optimize coating adhesion and performance.

This coating method offers several advantages, including the ability to create antimicrobial coatings for medical applications, improved control over coating properties, and the flexibility to use a wide range of coating materials. The continuous nature of the process, facilitated by the movement of the textile substrate web from the donor device to the receiving device, allows for high-volume production while maintaining consistent coating quality.

In one embodiment, the donor device is an unwinder roller or a donor container, and the receiving device is a rewinder roller or a recipient container. The textile substrate web is transported using diverter devices, particularly diverter rollers or diverter bars. This configuration allows for continuous and efficient movement of the textile substrate web through the coating process, enabling high-volume production while maintaining consistent coating quality.

Applying the coating solutions can be performed by immersion or spray-coating, each offering distinct advantages. In one embodiment, coating solution A is applied by immersing a section of the textile substrate web in a first coating bath containing the coating solution A. Immersion coating provides superior penetration into the textile structure, ensuring coating of individual fibers throughout the substrate thickness, and typically achieves for example 30-40% better coating uniformity compared to other methods. It also allows for precise control of exposure time and solution concentration, resulting in highly reproducible coating thickness. Similarly, coating solution B can be applied by immersing the section in a second coating bath containing coating solution B. Alternatively, coating solutions A and B can be applied using first and second spray-coating devices, respectively. Spray-coating offers advantages including reduced solution consumption (typically 15-25% less than immersion methods), minimized cross-contamination between coating stages, and the ability to selectively coat specific areas of the textile substrate. Spray-coating also enables more rapid processing, with coating application times being for example 40-60% shorter than comparable immersion processes, and facilitates easier adjustment of coating parameters for different substrate types. These complementary methods provide flexibility in the coating application process, allowing for optimization based on the specific requirements of the textile substrate and coating materials.

Removal of excessive residues is a critical step in ensuring clean and well-defined coating layers and is an essential prerequisite for generating monomolecular layers. In one embodiment, excessive residues of coating solution A are removed by immersing the section of the textile substrate web in a first rinsing bath. Similarly, excessive residues of coating solution B are removed by immersing the section in a second rinsing bath. Alternatively, the residues can be removed by flushing the section in first and second flushing devices for solutions A and B, respectively. These rinsing or flushing steps help maintain the integrity of each coating layer and prevent unwanted mixing of the coating solutions.

In one embodiment, excessive residues of coating solution A are removed by immersing the section of the textile substrate web in a first rinsing bath. Similarly, excessive residues of coating solution B are removed by immersing the section in a second rinsing bath. Alternatively, the residues can be removed by flushing the section in first and second flushing devices for solutions A and B, respectively. These rinsing or flushing steps help maintain the integrity of each coating layer and prevent unwanted mixing of the coating solutions.

The geometry of the coating baths plays a role in the coating process efficiency. In one embodiment, the textile substrate web has a web length and a web width, while the first and second coating baths each have a bath height, bath width, and bath length. The bath length is greater than the bath width and the web width of the textile substrate web, and the bath height is greater than the bath width.

This vertical orientation allows for efficient use of space while providing sufficient contact time between the substrate and the coating solutions. The narrow width of the baths helps to minimize the volume of coating solution required, potentially reducing waste and improving cost-efficiency while ensuring uniform coating application across the textile substrate web.

To further enhance the coating process, each of the coating baths and rinsing baths may include a diverter roller or a diverter bar. These components provide several advantages including precise control of the textile substrate web path through the coating and rinsing baths, minimization of web tension variations (typically maintaining tension for example within ±5% of target values), prevention of web sagging or folding that could lead to coating defects, and optimization of immersion time by controlling the exact path length within each bath. Additionally, the diverter rollers or bars enable a compact apparatus design by facilitating vertical or serpentine web paths, reducing the overall footprint by 30-40% compared to straight-line configurations, while ensuring consistent contact between the textile substrate and the coating solutions.

The diverter bar may be unpowered, which can simplify its integration within the coating and rinsing baths. This passive configuration allows for easier placement and maintenance within the confined space of a bath, as there is no need for complex drive mechanisms or seals to protect electrical components from the liquid environment. The unpowered design may also reduce the overall complexity and cost of the apparatus while still effectively guiding the textile substrate web through the coating process. In some cases, the smooth rotation of the diverter roller may be facilitated by the movement of the textile substrate itself, further enhancing the simplicity and reliability of the system.

These components help guide the textile substrate web through the baths, ensuring proper immersion and exposure to the coating and rinsing solutions.

The method may also include squeezing steps to remove excess coating solution. In one embodiment, the section of the textile substrate web is squeezed after applying coating solution A and/or coating solution B using a squeezing device. This step helps control the amount of coating solution retained on the textile substrate web, contributing to more uniform and controlled coating layers.

The duration of the coating application is an important parameter. In one embodiment, applying each of the coating solutions A and B to a section of the textile substrate web is performed between 3 seconds and 300 seconds, particularly between 10 seconds and 30 seconds, more preferably between 15 seconds and 25 seconds, and most preferably between 18 seconds and 22 seconds. These ranges allow for adequate coating application while maintaining efficient processing speeds.

To achieve the desired coating thickness and properties, the coating steps can be repeated. In one embodiment, the method includes repeating cycles comprising steps ii) to v) between 2 and 100 times, preferably between 4 and 30 times. This repetition allows for the creation of multiple alternating coating layers, enhancing the functional properties of the coated textile substrate.

The repetition of coating cycles can be performed in different ways, each offering distinct advantages for specific production scenarios. In one embodiment, the same first coating bath or first spray-coating device and the same second coating bath or second spray-coating device are used for each repeated cycle. This approach minimizes equipment requirements and floor space utilization, typically reducing capital investment compared to multiple-bath configurations. It also ensures consistent coating solution properties across all layers, as the same solutions are used throughout the process. However, this configuration may result in longer processing times, as the textile substrate must complete multiple passes through the same equipment. In the alternative embodiment, a series of at least two first coating baths or first spray-coating devices and a series of second coating baths or second spray-coating devices arranged in a row can be used. This linear arrangement enables significantly faster processing speeds-typically 2.5-3 times faster than the single-bath configuration-as the textile substrate makes a single pass through multiple sequential coating stations. The multi-bath configuration also allows for precise control of solution properties at each station, with the ability to adjust parameters such as concentration, temperature, or pH independently for each layer application. This approach is particularly advantageous for high-volume production environments where throughput is prioritized, though it requires greater initial investment in equipment and increased floor space. The selection between these approaches depends on specific production requirements including desired throughput, available space, capital constraints, and the degree of process control needed for the particular coating application.

The coating solutions used in the method are designed to create functional multilayer coatings. In one embodiment, the molecules contained in coating solution A and coating solution B can attract each other through electrostatic attraction. This property contributes to the formation of stable and well-defined coating layers.

Specifically, coating solution A may comprise Poly-L-arginine, a cationic polypeptide composed of L-arginine amino acid residues linked by peptide bonds, with the chemical formula (C6H14N4O2)n and characterized by positively charged guanidinium groups in the side chains, or Poly-ε-Lysine, a cationic homopolymer composed of L-lysine amino acid residues linked by peptide bonds between the carboxyl and epsilon-amino groups, with the chemical formula (C6H14N2O2)n and characterized by positively charged amino groups in the side chains, or a mixture thereof, while coating solution B comprises hyaluronan, an anionic, non-sulfated glycosaminoglycan distributed widely throughout connective, epithelial, and neural tissues, with the chemical formula (C14H21NO11)n and characterized by negatively charged carboxyl groups along its polymer chain.

These materials are known for their biocompatibility and potential antimicrobial properties, making them suitable for medical applications.

The coating process can result in the formation of monomolecular layers. In one embodiment, a monomolecular Poly-L-arginine layer is formed by applying coating solution A and removing excessive residues. Similarly, a monomolecular hyaluronan layer is formed by applying coating solution B and removing excessive residues. These ultra-thin layers allow for precise control over the coating structure and properties.

The term "monomolecular layer" may refer to substantially monomolecular layers, wherein the coating predominantly consists of a single molecular thickness. However, this term does not completely exclude the possibility that some molecules may occasionally overlap or lie on top of each other in certain areas of the coated surface. In some cases, minor deviations from a perfect single-molecule arrangement may occur due to factors such as surface irregularities, molecular interactions, or localized variations in the coating process. These occasional overlaps or multi-molecule regions may be present to a limited extent without significantly altering the overall characteristics and benefits of the monomolecular coating approach.

The method is applicable to various types of textile substrates. In one embodiment, the textile substrate web comprises one or more of woven textiles, knitted textiles, nonwoven textiles, and textile nets. This versatility allows for the production of coated textiles with a wide range of base material properties.

The dimensions and physical properties of the textile substrate web can vary based on the intended application. In one embodiment, the textile substrate web has a web width greater than 10 cm and smaller than 200 cm and/or a basis weight between 20 g/m² and 50 g/m².

Additionally, the textile substrate web may have a width of 110 cm, which is a typical dimension for industrial coating processes. This width allows for efficient use of standard coating equipment while providing sufficient material for various end-use applications. The 110 cm width may enable optimal utilization of coating solutions and facilitate uniform application across the substrate surface. In some cases, this width may also allow for the production of multiple smaller width products from a single coated web, potentially improving manufacturing efficiency.

Additionally, the textile substrate web may have an open-pored structure with a pore diameter of 0.1 to 5 mm. These characteristics can influence the coating process and in particular the properties of the final coated textile. The pore size significantly influences coating penetration and distribution throughout the textile structure. Smaller pores (0.1-1 mm) typically result in more surface-concentrated coatings with limited penetration into the substrate core, while larger pores (1-5 mm) allow deeper penetration of coating solutions, resulting in more uniform distribution throughout the textile thickness. Additionally, pore size affects fluid retention during the coating process, with smaller pores retaining more coating solution through capillary action, potentially increasing coating efficiency but requiring more thorough rinsing to remove excess material. The open-pored structure also maintains breathability in the final coated textile, with properly sized pores (typically 0.5-2 mm) preserving 70-80% of the original air permeability while still achieving effective antimicrobial coverage.

The coated textile substrate web produced by this method has various applications, particularly in the medical field. In one embodiment, a medical dressing or bandage comprises a textile layer cut out of the coated textile substrate web. The antimicrobial properties imparted by the coating can help prevent infections in wound care settings, with demonstrated efficacy against common pathogens including Staphylococcus aureus, Pseudomonas aeruginosa, and Candida albicans. Clinical testing has shown that these coated dressings can reduce bacterial colonization by for example 99.9% compared to uncoated controls. The multilayer coating structure enables controlled release of active agents over extended periods, typically maintaining therapeutic concentrations for 3-7 days depending on the specific coating formulation and number of layers applied. This sustained antimicrobial activity helps maintain a clean wound environment while reducing the frequency of dressing changes. Additionally, the coated textiles retain excellent moisture management properties, with water vapor transmission rates of for example 800-1200 g/m²/24h, which helps prevent maceration of surrounding tissue while maintaining optimal wound hydration. The flexibility of the coating process allows for customization of dressings for different wound types, including chronic wounds, surgical incisions, and burns, by adjusting the coating composition and layer structure to provide appropriate antimicrobial protection and healing support for each specific application.

This coating method offers several advantages, including precise control over layer composition and thickness, the ability to create complex multilayer structures, and the flexibility to use a wide range of coating materials. The continuous nature of the process allows for high-volume production while maintaining consistent coating quality. Additionally, the method enables the creation of functional coatings with specific properties tailored for medical applications, such as antimicrobial activity and biocompatibility.

The present invention provides several combined embodiments that implement any combinations of dependent claims, highlighting synergistic effects and additional advantages of these combinations.

One combined embodiment incorporates the squeezing and drying steps into a single process using a set of heated rollers. These heated rollers simultaneously apply pressure to remove excess coating solution and provide heat for drying. This combination offers synergistic benefits in terms of process efficiency and coating quality.

The integration of squeezing and drying steps into a single operation using heated rollers provides several advantages. First, the combined process reduces the overall processing time by eliminating the need for separate squeezing and drying stations. This can lead to increased production throughput and improved efficiency in the coating process.

The simultaneous application of pressure and heat allows for more effective removal of excess coating solution. The pressure applied by the rollers helps to mechanically squeeze out excess liquid, while the heat promotes rapid evaporation of any remaining moisture. This combination may result in more uniform coating thickness across the textile substrate web.

The heated rollers provide controlled and consistent heat application to the textile substrate web. This uniform heating may lead to improved coating adhesion and potentially enhance the formation of monomolecular layers. The controlled heat application may also help to set the coating more effectively, potentially improving its durability and performance.

By combining the squeezing and drying steps, the apparatus footprint may be reduced, as separate equipment for each process is no longer necessary. This can lead to more compact and space-efficient coating systems, which may be particularly advantageous in manufacturing environments with limited floor space.

The use of heated rollers for both squeezing and drying may also offer energy efficiency benefits. The heat used for drying is applied directly to the textile substrate web through conduction, which may be more energy-efficient than conventional convection or radiation drying methods.

Additionally, the combined squeezing and drying process using heated rollers may provide better control over the coating process. The pressure and temperature of the rollers can be precisely adjusted to accommodate different textile substrate materials, coating solutions, and desired coating thicknesses. This level of control may allow for fine-tuning of the coating process to achieve optimal results for various applications.

The integration of squeezing and drying may also help to minimize potential defects in the coating. By immediately drying the coating after squeezing, the risk of coating non-uniformities or imperfections that could occur during the transfer between separate squeezing and drying stations is reduced.

This combined embodiment may be particularly beneficial for creating highly uniform, multi-layer coatings on medical textiles. The precise control over pressure and temperature during the squeezing and drying process can be crucial for applications requiring specific antimicrobial properties or controlled release of active agents.

In practice, this combined embodiment may involve a series of heated roller pairs positioned after each coating bath. The textile substrate web passes through these heated rollers, which simultaneously squeeze out excess coating solution and dry the applied coating. The temperature and pressure of the heated rollers may be adjustable to optimize the process for different coating materials and textile substrates.

The synergistic effects of this combined embodiment extend to the overall coating process control. The integration of squeezing and drying steps allows for more precise and reproducible coating results, as the critical processes of excess solution removal and coating setting are performed in a single, controlled operation. This can be particularly valuable in the production of medical textiles where consistent performance is essential.

The invention encompasses various embodiments of an apparatus for coating a textile substrate web with multiple layers. In one embodiment, the apparatus includes a donor device for providing the textile substrate web, which may be an unwinder roll. The textile substrate web is guided through a series of processing stations arranged in sequence.

The apparatus may include multiple coating baths, each containing different coating solutions. For example, a first coating bath may contain coating solution A, while a second coating bath may contain coating solution B. Between the coating baths, rinsing baths filled with buffer solutions may be positioned to remove excess coating material.

Squeezing devices, such as pairs of rollers, may be incorporated after the coating baths to remove excess coating solution from the textile substrate web. These squeezing devices help control the amount of coating retained on the substrate.

Throughout the apparatus, multiple diverting rollers or bars may be positioned to guide the textile substrate web through the various processing stations. These diverting elements ensure proper web handling and exposure to the coating and rinsing solutions.

The apparatus may include a drying device, which could be an oven, infrared field, heat blower, or a combination thereof. This drying device helps set the applied coatings and prepares the substrate for subsequent processing or collection.

Web tension control and measurement devices may be incorporated at various points in the apparatus to maintain proper tension of the textile substrate web throughout the coating process.

At the end of the processing sequence, a receiving device, such as a rewinder roll, may be used to collect the coated textile substrate web.

In some embodiments, the apparatus may be configured with a vertical orientation of the processing baths. This arrangement allows for a compact design and efficient use of floor space in manufacturing environments.

The apparatus is designed to enable continuous processing of the textile substrate web as it moves from the donor device to the receiving device, allowing for the application of multiple coating layers in sequence. This continuous process facilitates high-volume production while maintaining consistent coating quality.

According to another possible embodiment, the coating process may be extended to include additional coating solutions containing different coating molecules. This approach may result in alternating sets of three or four different layers on the textile substrate web.

In this embodiment, the apparatus may include additional coating baths or spray-coating devices, each containing a unique coating solution. For example, a third coating bath may contain a coating solution C with coating molecules C, and a fourth coating bath may contain a coating solution D with coating molecules D. These additional coating stations may be integrated into the existing processing sequence.

The method may involve applying coating solution A, followed by coating solution B, then coating solution C, and optionally coating solution D, in a repeating cycle. Between each coating application, the excess coating solution may be removed, and the substrate may undergo a rinsing step to prepare it for the next coating layer.

This multi-layer approach may allow for the creation of more complex coating structures with enhanced functionality. For instance, each layer may contribute different properties to the final coated textile:
Layer A may provide antimicrobial or anti-biofilm properties
Layer B may enhance durability
Layer C may improve moisture management
Layer D may add a specific functional property such as UV protection or flame retardancy

The alternating nature of these multi-layer coatings may result in synergistic effects between the different coating molecules. For example, the interaction between adjacent layers may lead to improved overall coating stability or the creation of unique surface properties that are not achievable with single or dual-layer coatings.

The number of coating cycles may be adjusted to achieve the desired coating thickness and performance characteristics. The process may be repeated to create multiple sets of these alternating layers, with each set potentially consisting of three or four distinct layers.

This multi-layer coating approach may offer increased versatility in tailoring the properties of the coated textile substrate web for specific applications. It may allow for the development of advanced functional textiles with a combination of properties that are particularly well-suited for complex medical or technical applications.

The materials used in the coating process play a crucial role in determining the properties and performance of the coated textile substrate. This section provides a detailed description of the coating solutions, their components, and potential variants for different applications.

Coating solution A can comprise Poly-L-arginine, a cationic polyelectrolyte. Poly-L-arginine is a polymer of the amino acid arginine, which carries a positive charge at physiological pH due to its guanidinium group. In the context of this coating process, Poly-L-arginine with a chain length of 10 to 30 amino acids can be used. This specific chain length provides a balance between charge density and molecular mobility, facilitating efficient layer formation and interaction with the substrate and subsequent layers.

The concentration of Poly-L-arginine in coating solution A can be 0.1 to 2.0 mg/mL. This concentration range allows for effective layer formation while maintaining process efficiency and cost-effectiveness. Lower concentrations may result in incomplete layer coverage, while higher concentrations may lead to excessive material usage without significant improvement in coating quality.

Coating solution A can have a pH of 6.0 to 7.5. This pH range ensures that the Poly-L-arginine remains positively charged, promoting electrostatic interactions with the substrate and subsequent layers. The pH can be adjusted using appropriate buffers or dilute acids/bases as needed.

Coating solution B can comprise Hyaluronan or its sodium salt, sodium hyaluronate. Hyaluronan is a naturally occurring, anionic, non-sulfated glycosaminoglycan distributed widely throughout connective, epithelial, and neural tissues. In the context of this coating process, Hyaluronan serves as the anionic counterpart to the cationic Poly-L-arginine.

The Hyaluronan contained in coating solution B can have a molecular weight of 1.0 to 1.5 MDa. This molecular weight range provides a good balance between solution viscosity, layer formation properties, and biological activity. Higher molecular weight Hyaluronan may result in thicker layers but may also increase solution viscosity, potentially affecting the coating process.

The concentration of Hyaluronan in coating solution B can be 0.1 to 2.0 mg/mL, more preferably 0.5 mg/ml. This concentration range allows for effective layer formation while maintaining process efficiency. As with Poly-L-arginine, lower concentrations may result in incomplete layer coverage, while higher concentrations may not provide significant additional benefits.

Coating solution B can have a pH of 6.0 to 7.5. This pH range ensures that the Hyaluronan remains negatively charged, promoting electrostatic interactions with the previously deposited Poly-L-arginine layer. The pH can be adjusted using appropriate buffers or dilute acids/bases as needed.

The electrostatic interaction between molecule types A and B plays a key role in the formation of stable, in particular monomolecular, multilayer coatings on the textile substrate. Poly-L-arginine (molecule type A) carries a positive charge due to its guanidinium groups, while Hyaluronan (molecule type B) bears a negative charge from its carboxyl groups. This opposite charge characteristic allows for strong electrostatic attraction between the two types of molecules. The formation of essentially monomolecular layers is further promoted by the fact that the molecules A may repel each other due to their ionic character and that also the molecules B may repel each other due to their ionic character.

When the textile substrate is exposed to coating solution A, the positively charged Poly-L-arginine molecules may adsorb onto the substrate surface. Upon subsequent exposure to coating solution B, the negatively charged Hyaluronan molecules may be attracted to and bind with the previously deposited Poly-L-arginine layer. This alternating deposition process may result in the formation of self-assembled polyelectrolyte multilayers.

The strength of the electrostatic interaction may be influenced by factors such as pH, ionic strength of the coating solutions, and the molecular weight of the polyelectrolytes. By adjusting these parameters, the coating process may be optimized to achieve desired layer thickness, stability, and functional properties.

In some cases, the electrostatic interaction between Poly-L-arginine and hyaluronan may lead to the formation of interpolyelectrolyte complexes within the coating layers. These complexes may contribute to enhanced coating stability and may affect properties such as swelling behavior, permeability, and mechanical strength of the coated textile.

The alternating charge structure of the multilayer coating may also provide a mechanism for incorporating additional functional molecules or nanoparticles into the coating. For example, negatively charged antimicrobial agents may be integrated into the Poly-L-arginine layers, while positively charged bioactive compounds may be incorporated into the hyaluronan layers.

The solvent used for coating solutions A and B can preferably be a buffer, in particular a NaCl-Tris buffer. In one example, said NaCl-Tris buffer comprises 150 mM NaCl and 10 mM Tris at a pH 7.4. This buffer provides a controlled environment for the dissolution of the polyelectrolytes and helps maintain the desired pH of the coating solutions, ensuring consistent performance throughout the coating process.

The coating solutions can have a viscosity ranging from 0.25 cP to 1000 cP, with a preferred range of 0.30 cP to 2.00 cP. This viscosity range facilitates proper coating application and penetration into the textile structure. Lower viscosities may allow for better penetration into porous substrates, while higher viscosities may result in thicker coating layers.

The temperature of the coating baths can be maintained within a range of 10°C to 80°C, with a preferred range of 20°C to 60°C. This temperature range helps optimize the coating process for different coating materials and substrate types. Higher temperatures may increase the rate of layer formation but may also affect the stability of certain coating materials.

The textile substrate can have a porosity ranging from 10% to 90%, with a preferred range of 50% to 80%. This porosity range affects the interaction between the coating solutions and the substrate structure. Higher porosity may allow for better penetration of the coating solutions into the textile structure, while lower porosity may result in more surface-concentrated coatings.

The speed at which the textile substrate moves through the coating apparatus can range from 0.1 m/min to 100 m/min, with a preferred range of 1 m/min to 50 m/min. This speed range allows for efficient production while ensuring adequate coating application and processing time. Slower speeds may result in thicker coatings, while faster speeds may lead to thinner, more uniform coatings.

While Poly-L-arginine and hyaluronan are preferred for their biocompatibility and antimicrobial properties, other polyelectrolyte pairs may be used for specific applications. For example:
1. Chitosan and alginate: Chitosan is a cationic polysaccharide derived from chitin, while alginate is an anionic polysaccharide extracted from brown algae. This combination may be used for applications requiring enhanced wound healing properties.
2. Poly-ε-Lysine and hyaluronan: Poly-ε-Lysine provides a cationic component while hyaluronan serves as the anionic counterpart. This combination offers excellent biocompatibility and may be particularly beneficial for wound healing applications due to the moisture retention properties of hyaluronan combined with the antimicrobial characteristics of Poly-L-lysine.

The ionic strength of the coating solutions can be adjusted to fine-tune the layer formation process. Higher ionic strengths generally lead to thinner layers due to charge screening effects, while lower ionic strengths may result in thicker layers. The ionic strength can be adjusted by adding small amounts of salts such as sodium chloride or by modifying the buffer concentration.

For applications requiring enhanced antimicrobial activity, small amounts of antimicrobial agents such as silver nanoparticles or quaternary ammonium compounds may be incorporated into the coating solutions. These additives can provide additional protection against a broader spectrum of microorganisms.

The molecular weight of the polyelectrolytes can be varied to adjust the coating properties. Higher molecular weight polymers generally result in thicker layers and may provide enhanced durability, while lower molecular weight polymers may offer improved penetration into porous substrates.

The choice of materials, their concentrations, and process parameters can be optimized based on the specific requirements of the intended application, such as antimicrobial efficacy, biocompatibility, durability, or controlled release properties. For antimicrobial applications, the concentration of Poly-L-arginine in coating solution A may be adjusted between 0.1-2.0 mg/mL to achieve different levels of antimicrobial potency, with higher concentrations generally providing enhanced activity against gram-negative bacteria. Similarly, the molecular weight of hyaluronan in coating solution B can be selected between 0.8-1.8 MDa to modify release kinetics, with lower molecular weights typically resulting in faster release profiles. Process parameters such as immersion time (10-60 seconds), drying temperature (30-80°C), and interlayer rinsing duration (5-30 seconds) can be precisely calibrated to optimize coating uniformity and adhesion. The pH of coating solutions can be adjusted between 5.5-8.0 to modify the charge density and conformation of the polyelectrolytes, thereby influencing layer thickness and stability. For controlled drug release applications, therapeutic agents such as antibiotics, growth factors, or anti-inflammatory compounds can be incorporated at concentrations of 0.01-1.0% w/v, with their release profiles modulated by adjusting the number of coating layers (typically 4-20) and the ionic strength of the coating solutions (0.05-0.5 M NaCl). The flexibility of this coating method allows for a wide range of material combinations and process variations to achieve desired functional properties in the coated textile substrates.

The present invention solves the problems mentioned above by providing an apparatus for coating a textile substrate web with multilayer coatings, particularly antimicrobial multilayer coatings. The apparatus enables the application of alternating layers quickly, easily, and cost-effectively on an industrial scale.

An apparatus for coating a textile substrate web comprises a donor device for providing the textile substrate web, a first coating bath or a first spray-coating device, a first rinsing bath or a first flushing device, a second coating bath or a second spray-coating device, a second rinsing bath or a second flushing device, a drying device, and a receiving device for collecting the coated textile substrate web. The apparatus may optionally include a plurality of diverter rollers or diverter bars for guiding the textile substrate web through the process.

The apparatus includes a donor device, which may be an unwinder roll, for providing the textile substrate web. This donor device allows for continuous feeding of the textile substrate into the coating process.

A first coating bath or a first spray-coating device is included in the apparatus for applying a first coating solution to the textile substrate web. The first coating bath may be filled with a coating solution A. Alternatively, a first spray-coating device may be used to apply the coating solution A to the textile substrate web.

Following the first coating application, the apparatus includes a first rinsing bath or a first flushing device. This component serves to remove excess coating solution from the textile substrate web, ensuring a clean and uniform coating layer.

The apparatus further comprises a second coating bath or a second spray-coating device. The second coating bath may be filled with a coating solution B, or a second spray-coating device may be used to apply coating solution B to the textile substrate web. This allows for the application of a second, different coating layer to the textile substrate web.

After the second coating application, a second rinsing bath or a second flushing device is included in the apparatus. This component removes excess coating solution B from the textile substrate web, maintaining the integrity of the second coating layer.

A drying device is incorporated into the apparatus to set the applied coatings and prepare the substrate for subsequent processing or collection. The drying device may be a forced air dryer with adjustable temperature controls, an infrared panel heater with variable intensity settings, a contact heat roller, a vacuum drying chamber, a steam-heated cylinder, or a combination system employing multiple drying technologies in sequence. The choice of drying technology can be tailored to the specific requirements of the coating materials and textile substrate.

The apparatus includes a receiving device for collecting the coated textile substrate web. This receiving device may be a rewinder roll (12) or a recipient container, allowing for continuous collection of the processed textile substrate. The rewinder roll (12) is designed to maintain consistent tension during the winding process, typically incorporating adjustable torque control to accommodate different textile weights and coating thicknesses. This tension control is critical for preventing coating damage or deformation in the final collected product. The rewinder roll may include features such as variable speed control synchronized with the overall processing line speed, edge alignment mechanisms to ensure proper web tracking, and diameter sensors that automatically adjust winding parameters as the roll diameter increases. For applications requiring precise tension profiles, the rewinder roll may incorporate dancer rollers or load cells that provide feedback to the tension control system. The recipient container alternative may be employed for shorter production runs or when the coated textile requires a relaxed collection method to prevent stress on newly applied coatings.

Throughout the apparatus, a plurality of diverter rollers or diverter bars may optionally be included. These components guide the textile substrate web through the various processing stations, ensuring proper handling and exposure to the coating and rinsing solutions.

The apparatus may also include web tension control and/or measurement devices positioned at various points along the processing path. These devices help maintain proper tension of the textile substrate web throughout the coating process, contributing to uniform coating application and preventing damage to the substrate.

In some configurations, the apparatus may include additional coating baths filled with coating solution A or B, as well as additional rinsing baths. These extra processing stations allow for the application of multiple alternating layers of coatings, enhancing the functional properties of the coated textile substrate.

The apparatus is designed to enable continuous processing of the textile substrate web as it moves from the donor device to the receiving device. This continuous process facilitates high-volume production while maintaining consistent coating quality. The arrangement and sequence of components within the apparatus allow for the efficient application of multiple coating layers, rinsing steps, and drying, resulting in a coated textile substrate web with desired properties for various applications, including medical textiles with antimicrobial characteristics.

The present invention solves the problems mentioned above by providing a method for coating a medical textile substrate web with at least two layers of coating, particularly antimicrobial coatings, which are especially advantageous for medical applications, in a continuous and efficient manner.

The coatings disclosed herein may also exhibit an anti-biofilm effect. In some aspects, the multilayer coatings applied to the textile substrate web can inhibit or prevent the formation of biofilms on the coated surface. This anti-biofilm property may be particularly beneficial in medical applications where the prevention of microbial colonization and subsequent biofilm formation is crucial.

The anti-biofilm effect may be attributed to various factors, including the chemical composition of the coating layers, the surface properties imparted by the coating, and the potential incorporation of specific anti-biofilm agents. In some cases, the alternating layers of cationic and anionic polyelectrolytes may create a surface environment that is unfavorable for bacterial adhesion and biofilm initiation.

The Poly-L-arginine or Poly-ε-Lysine layers, for instance, may disrupt bacterial cell membranes and interfere with initial attachment processes. Meanwhile, the hyaluronan layers may provide a hydrophilic surface that resists protein adsorption and subsequent microbial colonization.

In some embodiments, the anti-biofilm effect may be enhanced by incorporating additional agents into the coating solutions. These may include quorum sensing inhibitors, enzymes that degrade extracellular polymeric substances, or nanoparticles with specific anti-biofilm properties. The multilayer coating approach allows for the strategic placement of these agents within the coating structure, potentially enabling sustained anti-biofilm activity over extended periods.

The anti-biofilm properties of the coated textiles may be particularly advantageous in applications such as wound dressings, or hospital textiles where preventing biofilm formation is essential for reducing the risk of infections and promoting healing. The ability to tailor the coating composition and structure allows for optimization of the anti-biofilm effect for specific use cases and target microorganisms.

The method for coating a medical textile substrate web involves providing the textile substrate web in a donor device and continuously moving the textile substrate web from the donor device to a receiving device. The process includes applying a coating solution A comprising coating molecules A to a section of the textile substrate web, followed by removing excessive residues of coating solution A. Subsequently, a coating solution B comprising coating molecules B is applied to the section of the textile substrate web, followed by removing excessive residues of coating solution B. The coating molecules A and coating molecules B are different. Optionally, the section of the textile substrate web may be dried using a drying device. Finally, the coated textile substrate web is collected in the receiving device.

The coating process follows a sequential series of steps. The process begins with providing a textile substrate on an unwinder roll. The substrate then undergoes immersion in a first coating bath containing coating solution A. Following this, the substrate is removed from the first coating bath and excess coating solution is removed.

The next step involves immersing the substrate in a first rinsing bath to remove any remaining excess coating solution A. After rinsing, the substrate is immersed in a second coating bath containing coating solution B. The process continues with removing the substrate from the second coating bath and removing excess coating solution B.

Following the application of coating solution B, the substrate is immersed in a second rinsing bath to remove any remaining excess coating solution B. After the coating and rinsing steps are completed, the substrate may undergo an optional drying step using a drying device.

The final step involves collecting the coated substrate on a rewinder roll. This process illustrates a continuous workflow where the substrate moves through multiple coating and rinsing stages in sequence, allowing for the application of different coating solutions with intermediate rinsing steps.

The method may be repeated to apply additional layers of coating, with the number of repetitions determined by the desired coating thickness and properties. This process allows for the creation of multilayer coatings with alternating compositions, which may enhance the functional properties of the coated textile substrate.

This coating method offers several advantages, including precise control over layer composition and thickness, the ability to create complex multilayer structures, and the flexibility to use a wide range of coating materials. The continuous nature of the process allows for high-volume production while maintaining consistent coating quality.

Features of any of the examples or embodiments outlined above may be combined to create additional examples or embodiments without losing the intended effect. It should be understood that the description of an embodiment or example provided above is by way of example only, and various modifications could be made by one skilled in the art. Furthermore, one skilled in the art will recognise that numerous further modifications and combinations of various aspects are possible. Accordingly, the described aspects are intended to encompass all such alterations, modifications, and variations that fall within the scope of the appended claims.

### FIGURES

Non-limiting and non-exhaustive examples are described with reference to the following figures. The figures illustrate various aspects of an apparatus and method for coating a textile substrate web with multiple layers. The figures show:
FIG. 1 Schematic side view of an apparatus (100) for coating a textile substrate web (15). It shows a complete processing sequence from an unwinder roll (1) to a rewinder roll (12), including multiple coating baths, rinsing baths, a squeezing device, and a drying unit. Diverting rollers guide the substrate throughout the process.
FIG. 2 Schematic side view of an alternative configuration of the coating apparatus (200). It illustrates a streamlined process with fewer coating stages compared to FIG. 1, detailing the path of the textile substrate web through various treatment baths and highlighting the strategic placement of diverting rollers.
FIG. 3 Schematic side view of a simplified version of the coating apparatus (300). It focuses on the core components of the coating process, showing the arrangement of coating and rinsing baths, as well as the crucial role of diverting rollers in maintaining proper substrate tension and guidance.
FIG. 4 Flowchart of the method for coating a textile substrate. It delineates each step of the process from unwinding to rewinding, providing a clear, sequential representation of the coating procedure and emphasizing the order and significance of each treatment stage.
FIG. 5 Orthogonal view of a coating bath illustrating the dimensional parameters.
FIG. 6 Orthogonal view of a textile substrate web illustrating the dimensional parameters. The figure shows the web length (wl) and web width (ww) of the textile substrate web.

### DETAILED DESCRIPTION OF THE FIGURES

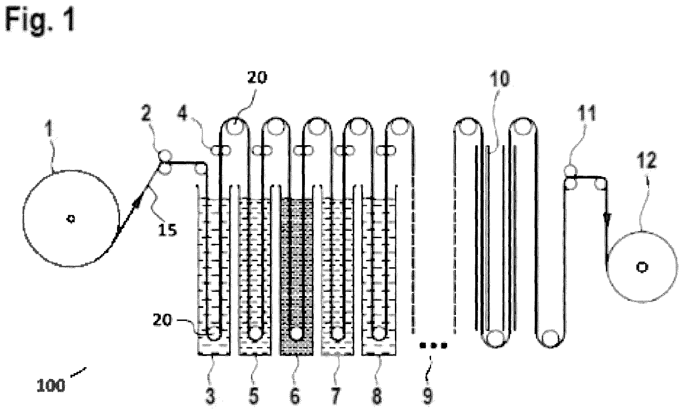

FIG. 1 provides a detailed schematic side view of an embodiment of the apparatus (100) for coating a textile substrate web (15), illustrating the complete processing sequence from unwinder roll (1) to rewinder roll (12). The figure showcases multiple processing stations including a first coating bath (3) containing coating solution A, a squeezing device (4) with a pair of rollers, a first rinsing bath (5) filled with buffer, a second coating bath (6) containing coating solution B, a second rinsing bath (7) filled with buffer, a further coating bath (8) filled with coating solution A, and additional coating and rinsing baths (9). Accordingly reference (9) may represent a series of multiple baths arranged in a row. This configuration allows for the application of additional coating layers and subsequent rinsing steps beyond the initial coating sequence. The number of baths included in this series may vary depending on the desired coating thickness, complexity of the multilayer structure, or specific requirements of the final coated textile product. By incorporating multiple baths in succession, the apparatus can facilitate the creation of more complex coating structures with a higher number of alternating layers without the need for rewinding or reprocessing the textile substrate web. This arrangement may enhance production efficiency and enable the creation of coatings with advanced functional properties through the strategic layering of different coating solutions.

The apparatus also includes web tension control and/or measurement devices (2, 11) positioned after the unwinder roll (1) and before the rewinder roll (12), respectively. A drying device (10), which may be an oven, infrared field, heat blower or combination thereof, is positioned near the end of the processing sequence. Throughout the apparatus, multiple diverting rollers or bars (20) are strategically positioned to guide the textile substrate web (15) through each processing station, ensuring proper web handling and exposure to the various coating and rinsing solutions.

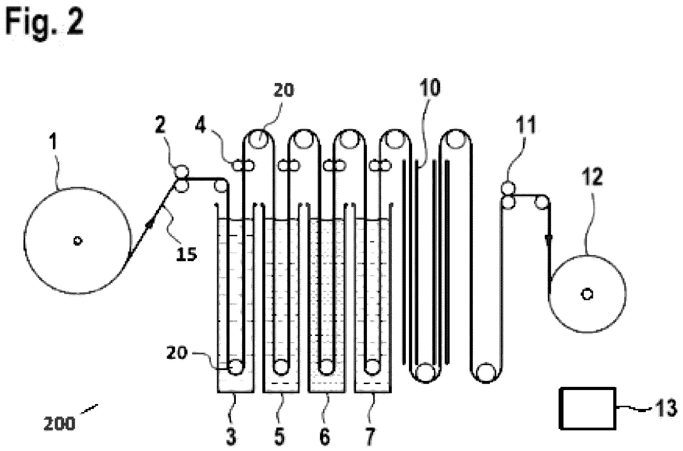

FIG. 2 presents a comprehensive schematic side view of an alternative configuration of the coating apparatus (200), emphasizing a streamlined process with fewer coating stages compared to FIG. 1. This figure meticulously details the path of the textile substrate web (15) through various treatment baths, beginning at the unwinder roll (1) and terminating at the rewinder roll (12). The textile substrate web (15) first passes through a web tension control and/or measurement device (2) before entering the first coating bath (3) containing coating solution A. After exiting the first coating bath (3), the web proceeds through a squeezing device (4) equipped with a pair of rollers that remove excess coating solution. The web then continues through a first rinsing bath (5) filled with buffer solution, followed by a second coating bath (6) containing coating solution B, and subsequently a second rinsing bath (7) filled with buffer solution. Before collection on the rewinder roll (12), the web passes through a drying device (10), which may be an oven, infrared field, heat blower, or combination thereof, and another web tension control and/or measurement device (11). The figure highlights the strategic placement of multiple diverting rollers or bars (20) throughout the apparatus, which are positioned within each bath and at critical transition points to ensure optimal substrate handling, maintain proper web tension, and facilitate smooth directional changes as the web navigates through the coating process. A controller (13) is also shown, which coordinates the operation of the various components to ensure synchronized processing.

The coating process may be repeated multiple times to build up additional layers on the textile substrate web. In some aspects, the method may involve cycling the substrate through the coating and rinsing steps several times to achieve the desired number of alternating layers. The number of repetitions may be adjusted based on the specific requirements of the final coated textile product.

By repeating the process, multiple bilayers consisting of coating A and coating B may be deposited on the substrate surface. In some cases, this repetition may allow for the creation of complex multilayer structures with enhanced functional properties. The ability to control the number of coating cycles may provide flexibility in tailoring the thickness, composition, and performance characteristics of the final coated textile.

The repeated application of alternating layers may contribute to improved coating durability, as each additional layer may reinforce the overall coating structure. In some implementations, the multilayer coating formed through repeated cycles may exhibit synergistic effects between the different coating materials, potentially leading to enhanced antimicrobial efficacy, improved barrier properties, or other desired functionalities.

The apparatus may be configured to accommodate multiple passes of the textile substrate web through the coating and rinsing stations. In some configurations, this may involve redirecting the web back through the same processing sequence, while in other arrangements (see Figure 1), additional coating and rinsing baths may be incorporated in series to facilitate continuous multilayer deposition without the need for rewinding.

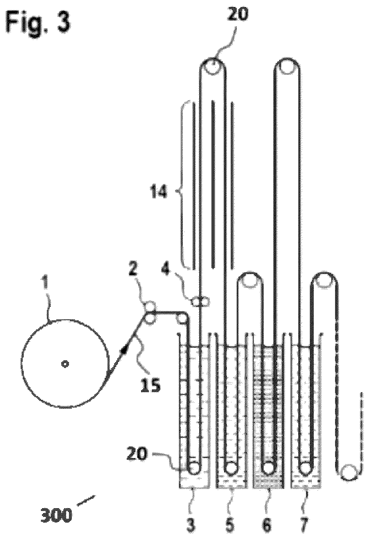

FIG. 3 offers an in-depth schematic side view of a simplified version of the coating apparatus (300), focusing on the core components essential for the basic multilayer coating process. This figure elucidates the arrangement of coating and rinsing baths in a compact configuration, beginning with an unwinder roll (1) that serves as the donor device for providing the textile substrate web (15). After passing through a web tension control and/or measurement device (2), the textile substrate web (15) enters a first coating bath (3) containing coating solution A, where it is immersed to apply the initial coating layer. Upon exiting the first coating bath (3), the web proceeds through a squeezing device (4) with a pair of rollers that apply pressure to remove excess coating solution, ensuring a more uniform coating thickness. The web then continues to a first rinsing bath (5) filled with buffer solution, which removes any residual unbound coating material from the substrate surface. Following this rinsing step, the web enters a second coating bath (6) containing coating solution B, where a second, different coating layer is applied. After the second coating application, the web passes through a second rinsing bath (7) filled with buffer solution to remove excess coating solution B. Throughout this processing sequence, the figure illustrates the crucial role of multiple diverting rollers or bars (20) strategically positioned within each bath and at transition points between processing stations. These diverting elements serve multiple critical functions: they maintain proper substrate tension to prevent sagging or wrinkling, guide the web along a predetermined path through each processing bath, ensure consistent immersion depth in the coating and rinsing solutions, facilitate directional changes with minimal stress on the substrate, and enable a compact apparatus design by allowing vertical or serpentine web paths. The precise positioning of these diverting rollers or bars (20) is essential for achieving uniform coating application and maintaining the integrity of the textile substrate throughout the coating process.

Additionally, a drying device (14) located between the first coating bath and first rinsing bath may be included to provide intermediate drying when needed.

The drying device (14) located between the first coating bath (3) and first rinsing bath (5) may serve several important functions in the coating process. This intermediate drying step may help control the moisture content of the textile substrate web (15) after the initial coating application. By partially or fully drying the substrate before it enters the first rinsing bath (5), the drying device (14) may enhance the stability of the first coating layer and potentially improve its adhesion to the textile surface.

In some cases, the drying device (14) may also help prevent excessive dilution of the rinsing bath by removing excess coating solution from the substrate. This may lead to more efficient use of the rinsing solution and potentially reduce contamination between processing stages. The presence of this drying device may allow for greater flexibility in adjusting process parameters, as it provides an additional point of control in managing the coating application and rinsing steps.

The drying device (14) may utilize various technologies such as forced air, infrared heating, or contact heat rollers, depending on the specific requirements of the coating materials and textile substrate. The intensity and duration of the drying process at this stage may be adjusted to optimize the overall coating performance and efficiency of the subsequent rinsing step.

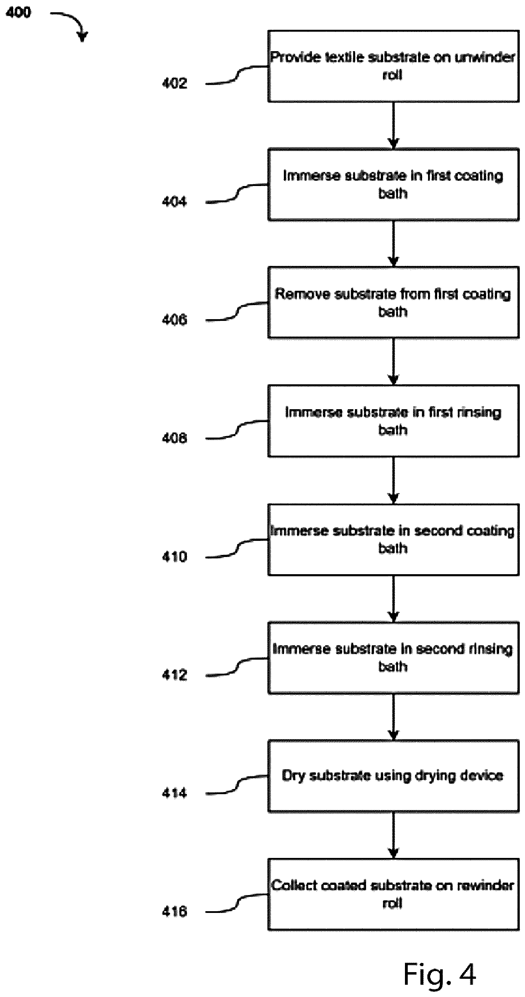

FIG. 4 illustrates a detailed flowchart of the method for coating a textile substrate, delineating each step of the process from unwinding to rewinding. This figure provides a clear, sequential representation of the coating procedure, emphasizing the order and significance of each treatment stage in the overall coating process.

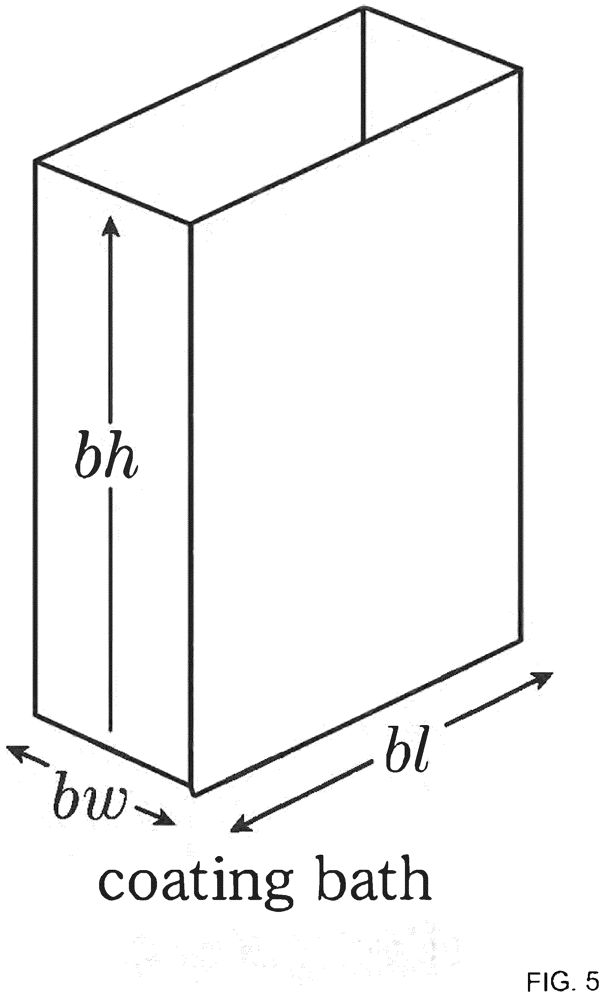

FIG. 5 illustrates an orthogonal view of a coating bath. The figure shows the dimensional parameters of the bath structure, including the bath height (bh), bath width (bw), and bath length (bl). The bath is depicted as a rectangular container with an open top.

The bath length (bl) extends along the horizontal axis and represents the longest dimension of the bath. The bath width (bw) represents the shorter horizontal dimension of the bath. The bath height (bh) represents the vertical dimension of the bath from its base to its upper edge.

The dimensions are arranged such that the bath length (bl) is greater than the bath width (bw), and the bath height (bh) is greater than the bath width (bw). These proportions create a vertically-oriented, elongated bath structure suitable for immersing textile substrates during the coating process.

The figure includes dimensional arrows and labels indicating the bath height (bh), bath width (bw), and bath length (bl), providing a clear representation of the bath's proportional relationships and spatial configuration.

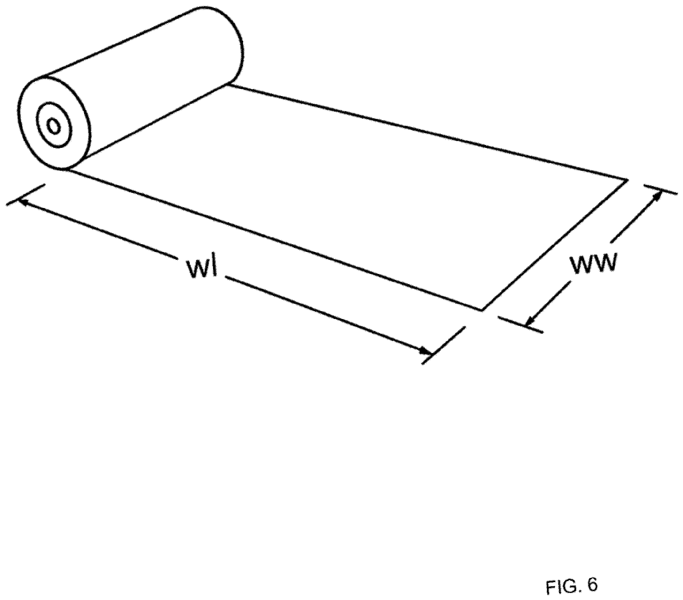

FIG. 6 illustrates an orthogonal view of a textile substrate web. The figure shows the dimensional parameters of the web, including the web length (wl) and web width (ww). The textile substrate web is depicted as a rectangular shape with a roll at one end.

The web length (wl) extends along the horizontal axis and represents the longer dimension of the web. The web width (ww) represents the shorter horizontal dimension of the web, measured perpendicular to the web length. A typical web width is 110 cm. Typical web length are in the range of 50 to 200 m.

The figure includes dimensional arrows and labels indicating the web length (wl) and web width (ww), providing a clear representation of the web's dimensional parameters. The roll portion at one end may indicate how the textile substrate web can be stored and dispensed during processing.

This orthogonal view helps visualize the key dimensions of the textile substrate web that are relevant to the coating process. Understanding these parameters may be important for optimizing the coating apparatus design and ensuring proper handling of the substrate throughout the coating procedure.

Common reference numerals are used throughout the figures to indicate similar features.

## Claims

1. A method for coating a medical textile substrate web (15) with at least two layers of coating, in particular with at least two layers of an antimicrobial effective coating, the method comprising:
step i) providing the textile substrate web (15) in a donor device (1);
step ii) applying a coating solution A comprising coating molecules A to a section of the textile substrate web (15);
step iii) removing excessive residues of coating solution A from the section of the textile substrate web (15);
step iv) applying a coating solution B comprising coating molecules B to the section of the textile substrate web (15);
step v) removing excessive residues of coating solution B from the section of the textile substrate web (15);
step vi) optionally drying the section of the textile substrate web (15) using a drying device (10);
step vii) collecting the textile substrate web (15) in a receiving device (12);
wherein the textile substrate web (15) is transported by continuously moving the textile substrate web (15) from the donor device (1) to the receiving device (12),
and wherein the coating molecules A and the coating molecules B are different.

2. The method according to claim 1, wherein the donor device is an unwinder roller (1) or a donor container and/or the receiving device is a rewinder roller (12) or a recipient container, and wherein the textile substrate web (15) is transported by the use of diverter devices, in particular diverter rollers or diverter bars.

3. The method according to claim 1 or 2, wherein applying the coating solution A to the section of the textile substrate web (15) in step ii) is performed by immersing the section of the textile substrate web (15) in a first coating bath (3) containing the coating solution A or by applying the coating solution A to the section of the textile substrate web (15) by the use of a first spray-coating device, and/or wherein applying the coating solution B to the section of the textile substrate web (15) in step iv) is performed by immersing the section of the textile substrate web (15) in a second coating bath (6) containing the coating solution B or by applying the coating solution B to the section of the textile substrate web (15) by the use of a second spray-coating device.

4. The method according to any one of claims 1 to 3, wherein removing excessive residues of the coating solution A from the section of the textile substrate web (15) in step iii) is performed by immersing the section of the textile substrate web (15) in a first rinsing bath (5) or by flushing the section of the textile substrate web (15) in a first flushing device, and/or wherein removing excessive residues of the coating solution B from the section of the textile substrate web (15) in step v) is performed by immersing the section of the textile substrate web (15) in a second rinsing bath (7) or by flushing the section of the textile substrate web (15) in a second flushing device.

5. The method according to any one of claims 1 to 4, wherein applying the coating solution A to the section of the textile substrate web (15) in step ii) is performed by immersing the section of the textile substrate web (15) in a first coating bath (3) containing the coating solution A, and wherein removing excessive residues of the coating solution A from the section of the textile substrate web (15) in step iii) is performed by immersing the section of the textile substrate web (15) in a first rinsing bath (5),
and wherein applying the coating solution B to the section of the textile substrate web (15) in step iv) is performed by immersing the section of the textile substrate web (15) in a second coating bath (6) containing the coating solution B,
and wherein removing excessive residues of the coating solution B from the section of the textile substrate web (15) in step v) is performed by immersing the section of the textile substrate web (15) in a second rinsing bath (7),
and wherein the section of the textile substrate web (15) is dried using a drying device (10).

6. The method according to claim 5, wherein each of the coating baths (3, 6) and each of the rinsing baths (5, 7) includes a diverter roller (20) or a diverter bar (20).

7. The method according to claims 5 or 6, wherein the textile substrate web (15) has a web length (wl) and a web width (ww), wherein the first coating bath (3) and the second coating bath (6) each have a bath height (bh), a bath width (bw), and a bath length (bl), and wherein the bath length (bl) is greater than a bath width (bw) and wherein the bath length (bl) is greater than a web width (ww) of the textile substrate web (15), and the bath height (bh) is greater than the bath width (bw).

8. The method according to any one of claims 1 to 7, further comprising squeezing the section of the textile substrate web (15) after applying the coating solution A to the section of the textile substrate web (15) using a squeezing device (4), and/or
further comprising squeezing the section of the textile substrate web (15) after applying the coating solution B to the section of the textile substrate web (15) using a squeezing device (4).

9. The method according to any one of claims 1 to 8, wherein applying each of the coating solution A and coating solution B to the section of the textile substrate web (15) is performed between 3 seconds and 300 seconds, in particular between 10 seconds and 30 seconds.

10. The method according to any one of claims 1 to 9, further comprising repeating cycles comprising the steps ii) to v) between 2 and 100 times, preferably between 4 and 30 times, to obtain between 4 and 200, preferably between 8 and 60 alternating coating layers on the textile substrate web (15).

11. The method according to claim 10, wherein the repetition of the cycles ii) to v) is performed by one of:
using the same first coating bath (3) or first spray-coating device as well as the same second coating bath (6) or second spray-coating device for each of the repeated cycles of application; or
by using a series of at least two first coating bathes (3) or first spray-coating devices as well as a series of second coating bathes (6) or second spray-coating devices arranged in a row.

12. The method according to any one of claims 1 to 11, wherein the molecules contained in the coating solution A and the molecules contained in the coating solution B can attract each other through electrostatic attraction.

13. The method according to any one of claims 1 to 12, wherein the coating solution A comprises Poly-L-arginine or Poly-ε-Lysine or a mixture of Poly-L-arginine and Poly-ε-Lysine, and wherein the coating solution B comprises hyaluronan.

14. The method according to claim 13, wherein a monomolecular Poly-L-arginine layer is formed by applying the coating solution A to the section of the textile substrate web (15) and removing excessive residues of coating solution A from the section of the textile substrate web (15); and
wherein a monomolecular hyaluronan layer is formed by applying the coating solution B to the section of the textile substrate web (15) and removing excessive residues of coating solution B from the section of the textile substrate web (15).

15. The method according to any one of claims 1 to 14, wherein the textile substrate web (15) comprises one or more of woven textiles, knitted textiles, nonwoven textiles, and textile nets.

16. The method according to any one of claims 1 to 15, wherein the textile substrate web (15) has a web width greater than 10 cm and smaller than 200 cm and/or a basis weight between 20 g/m² and 50 g/m².

17. The method according to any one of claims 1 to 16, wherein the textile substrate web (15) has an open-pored structure with a pore diameter of 0.1 to 5 mm.

18. An apparatus (100, 200, 300) for coating a textile substrate web (15), the apparatus comprising:
a donor device (1) for providing the textile substrate web (15);
a first coating bath (3) or a first spray-coating device;
a first rinsing bath (5) or a first flushing device;
a second coating bath (6) or a second spray-coating device;
a second rinsing bath (7) or a second flushing device;
a drying device (10);
a receiving device (12) for collecting the coated textile substrate web (15); and
optionally a plurality of diverter rollers (20) or diverter bars (20) for guiding the textile substrate web (15) through the process,
wherein the apparatus (100, 200, 300) is configured to perform the method according to any one of claims 1 to 17.

19. A coated textile substrate web (15) produced by the method according to any one of claims 1 to 17.

20. A medical dressing or bandage comprising a textile layer, which textile layer is cut out of a coated textile substrate web (15) according to claim 19.
